# EUROPEAN PATENT SPECIFICATION

(11) **EP 2 420 227 B1**
(45) Date of publication and mention of the grant of the patent: **22.01.2014**
(21) Application number: 10764096.3
(22) Date of filing: 14.04.2010
(51) Int. Cl.: A61K 31/44, A61K 9/00, A61K 9/16, A61K 9/50, A61P 35/00

(54) **TARGETED SUSTAINED-RELEASE MICROSPHERE OF VASCULAR OCCLUSIVE AGENT CONTAINING SODIUM ALGINATE AND SORAFENIB, PRODUCTION METHOD AND USE THEREOF**
GEZIELTE RETARD-MIKROKÜGELCHEN MIT EINEM GEFÄSSOKKLUSIONSMITTEL MIT NATRIUMALGINAT UND SORAFENIB, HERSTELLUNGSVERFAHREN UND ANWENDUNG
MICROSPHÈRE À LIBÉRATION PROLONGÉE CIBLÉE D'UN AGENT D'OCCLUSION VASCULAIRE À BASE D'ALGINATE DE SODIUM ET DE SORAFÉNIB, MÉTHODE DE PRODUCTION ET UTILISATION

(30) Priority: 16.04.2009 CN 200910082449
(43) Date of publication of application: 22.02.2012
(73) Proprietor: Beijing Shengyiyao Science & Technology Development Co., Ltd, Xicheng District Beijing 100088 (CN)
(72) Inventor: LI, Xinjian, Beijing 100088 (CN); HONG, Hong, Beijing 100088 (CN); LU, Ge, Beijing 100088 (CN)
(74) Representative: Bösl, Raphael Konrad
(86) International application number: PCT/CN2010/071749
(87) International publication number: WO 2010/118683

(56) References cited:
- WO-A1-2009/111648
- WO-A2-2005/062810
- CN-A- 1 633 285
- CN-A- 101 301 470
- US-A1- 2007 031 338
- US-A1- 2008 020 052
- DATABASE WPI Week 200929 Thomson Scientific, London, GB; AN 2009-E28623 XP002686598, -& CN 101 336 890 A (JINAN JIFU MEDICINE SCI&TECHNOLOGY LTD) 7 January 2009 (2009-01-07)
- FURUSE JUNJI: "Sorafenib for the treatment of unresectable hepatocellular carcinoma.", BIOLOGICS : TARGETS & THERAPY DEC 2008 LNKD- PUBMED:19707458, vol. 2, no. 4, December 2008 (2008-12), pages 779-788, XP002686599, ISSN: 1177-5475

## Description

### Technical Field

The present invention relates to a microsphere vascular embolizing agent comprising anti-tumor drug, the preparation method and the use thereof. The present invention especially relates to a targeted sustained-release sodium alginate microsphere vascular embolizing agent containing sorafenib, the preparation method and the use thereof.

### Background Technologies

Sorafenib is a novel diaryl urea, under the chemical name of 4-{4-[3-(4-chloro-3-trifluoro-phenyl)-ureido]-phenoxyl}-pyridine-2-carboxylic methylamine, whose molecular weight is 464.8 g/mol. The sorafenib used in clinic is its tosylate salt. The molecular formula of sorafenib tosylate is C₂₁H₁₆C₁F₃N₄O₃·C₇H₈O₃S, the molecular weight is 637.0 g/mol and the chemical formula is shown below:

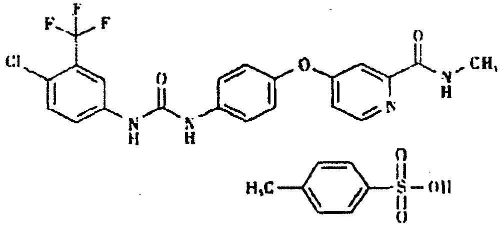

The melting point of sorafenib tosylate is 225-235 °C, and is a kind of tasteless solid, intermediate between white and brown. It is heat-stable and nonabsorbent; its solubility is low in aqueous solution and becomes higher a little bit under the strongly acid condition. It is slightly soluble in alcohol but can be dissolved in polyethylene glycerol 400. Sorafenib is a multi-target tumor-targeting therapeutic drug, which was co-developed by Bayer and ONYX during their collaboration since 1994. It is the first tumor-targeting drug approved by the U.S. Food and Drug Administration (FDA), for the treatment of metastatic renal cancer. Sorafenib was officially approved by U.S. FDA in December 2005 for use in the treatment of advanced renal cancer and it therefore became the first commercially available oral multikinase inhibitor. It was authorized to be marketed in China in End November of 2006.

Sorafenib acts as tyrosine kinase inhibitor, angiogenesis inhibitor, as well as vascular endothelial growth inhibitor. The survival, growth and metastasis of tumor are dependent on the effective cell proliferation and angiogenesis of tumor. Ras (GTP binding protein)/Raf signal transduction is a key pathway involved in tumor cell proliferation and angiogenesis. Raf is a serine/threonine (Ser/Thr) protein kinase and is the downstream effector enzyme of Ras protein. Once Raf is activated, mitogen-activated protein kinase (MEK) 1 and 2 are both triggered, which in turn cause extracellular signal-regulated kinase (ERK) 1 and 2 to be activated by phosphorylation and then translocated to nucleus. Transcription initiation and translation activation pathways are therefore triggered, resulting in cell proliferation. So this signal transduction pathway plays a direct role in regulating tumorigenesis and tumor developement in various human tumor tissues. On one hand, sorafenib inhibits RAS/RAF/MEK/ERK signal transduction pathway by inhibiting the activity of RAF so as to inhibit tumor cell growth directly. On the other hand, sorafenib interrupts neovascularization of tumor and also cuts off the nutrition supply of tumor cells by inhibiting the activity of several tyrosine kinase receptors involved in neovascularization and development of tumor, including vascular endothelial growth factor receptor 2 (VEGFR-2), REGFR-3, platelet derived growth factor receptor β (PDGFR-β) and proto-oncogene C-kit, so as to inhibit the growth of tumor cells indirectly. Thus, sorafenib has a dual anti-tumor function.

Sorafenib is mainly metabolized in liver through CYP3A4 enzyme oxidative metabolism and UGTIA9 glucuronic acid metabolism. Eight of metabolic products have been identified and five of them have been measured in blood. The predominant metabolic product pyridine N-Oxide, accounting for 9%∼16% of all metabolic products, plays a similar role to sorafenib *in vitro.* When orally taking a single dose of a solution containing 100 mg sorafenib, 77% of sorafenib is excreted in the faeces within 14 days, where 15% is excreted unchanged; while 19% of sorafenib is excreted as glucuronic acid metabolic products in urine. Sorafenib is the first drug allowed to enter clinical trials among congeneric compounds. The preliminary results of the clinical studies suggest that sorafenib has anti-tumor effects on solid tumors such as renal cancer, liver cancer, melanoma, non-small cell lung cancer, gastric cancer, ovarian cancer.

The common side effects of sorafenib include skin redness, rash, itching, hair loss or patchy hair loss, frequent diarrhea and/or enterokinesia relaxation, nausea or vomiting, oral ulcer, fatigue, appetite loss (decrease), high blood pressure, hand-foot syndrome. Among the common side effects of sorafenib, dermal toxicity and gastrointestinal responses are the main reasons for decreasing dosage or halting medication. During the treatment with oral administration of sorafenib, the most common side effects are the ones caused in gastrointestinal track, wherein 95% is gastrointestinal response, 58% is diarrhea, 30% is nausea, 24% is vomiting, and 47% is indigestion accompanied by appetite loss.

The potential side effects announced by the U.S. FDA include birth defect or death of fetus. Thus, any methods of birth control should be adopted for both male and female during the treatment and for two weeks after stopping taking sorafenib. The potential side effects such as redness, pain, swelling or blistering in palm and thenar may be observed as well. Blood pressure should be checked every week during the first six weeks of the treatment. If high blood pressure is caused while taking the medication, it should be treated in time. If patient has any potential heart problems, doctors should be informed before medication, since some side effects in heart may be caused during the treatment.

A number of genes and kinases are involved in tumorigenesis and tumor development, so targeted therapy has become one of the hottest research fields nowadays. Based on the further disclosure of the molecular biology mechanism of tumorigenesis, sorafenib was developed successfully as a novel drug possessing a unique multi-target anti-tumor activity. Its successful application in clinic inaugurated a new era of biological targeted therapy on tumors. In the aspects of the mechanism of action as well as the clinical trial results, sorafenib is different from chemotherapeutic drugs as it works mainly by inhibiting growth of tumor cells instead of via cytotoxic effects. The clinical trial results have verified that the second-line treatment of advanced renal cell carcinoma with sorafenib can prolong PFS, OS and TTP markedly. There are no doubts that the biggest concerns at the clinical trial stage nowadays are how to further enhance therapeutic effects of sorafenib and how to seek available markers to predict its therapeutic effects. Sorafenib is a novel multi-kinase inhibitor, which can inhibit not only RAF-MEK-ERK pathway but also tyrosine kinase receptors so as to result in the inhibition of tumor growth and angiogenesis. The Phase I clinical trial showed that it is tolerated and effective to take 400 mg orally twice a day and the most common side effects caused are diarrhea and skin lesion. The Phase II clinical trial suggested that sorafenib has anti-tumor activities on liver cancer and renal cancer, respectively. The Phase III clinical trial on advanced renal cancer has proved that the tumor in most of patients was shrunk markedly and the median survival time was prolonged dramatically too. Currently, a number of Phase III clinical trials, such as the Phase III clinical trial of treating liver cancer with sorafenib in China, are still underway. Thus, it is believed that more inspiring outcomes will come up and new hope of treating tumors may also arise. While holding up hopes, it should be noted that a great many problems are pending to be resolved, for example, the fact that it is difficult for drugs to penetrate tumor tissues during medication. Further clinical trials should be performed to overcome the difficulty for drugs of reaching tumor tissues and resolve the problem of treating with low concentration.

A Phase II study carried out by Ghassan *et al.* on the treatment of liver cancer with sorafenib has shown that the monotherapy with sorafenib has some therapeutic effects on liver cancer. Although the researchers deem that the monotherapy with sorafenib is not very effective, the effect of sorafenib is close to that of combined chemotherapy. The mechanism of action as well as the lower toxicity of sorafenib also allows it to be combined with other anti-cancer drugs to further enhance therapeutic effects. This study is a multi-center Phase III clinical trial in the Asian-Pacific area focusing on middle or advanced liver cancer cases where the patients are unable or unwilling to undergo surgery. As the nosogenesis of liver cancer is being disclosed and new molecular targeted drugs are being studied, patients suffering from advanced liver cancer may be offered an opportunity to receive targeted therapy. Most of liver cancer is descended from hepatocirrhosis so the patients with liver dysfunction and in poor physical condition benefit very little from chemotherapy and radiotherapy. Rapid advances in molecular targeted drugs provide alternative methods to treat liver cancer. In a word, molecular targeted therapy with good targets and low toxicity shows a broad prospect of treating advanced liver cancer. Thus, this kind of drug is probably one of the most potential and promising methods to treat liver cancer in the future.

Primary carcinoma of liver is an extremely malignant tumor. Although excision may be the first therapy, around 70% of patients are already in the middle or terminal stage while diagnosed. At that time, broad lesions or even metastasis has already happened, which is usually accompanied by hepatocirrhosis, so that the tumor cannot be excised surgically in those cases. Transcatheter arterial chemoembolization (TACE) is one of the key therapies to treat middle and advanced liver cancer and it works by utilizing chemotherapeutic agents as well as embolism. Since 90%-95% of the blood supply of liver cancer is from hepatic artery, infusion of chemotherapeutic agents and embolism through hepatic artery may cause ischemic necrosis of tumors by blocking their blood supply, which allows high-dose agents work particularly on tumors for a longer time so as to improve therapeutic effects eventually. Additionally, treating with TACE before liver cancer surgery may lead to necrosis, absorption and fibrosis of tumor tissues and formation of thick fibrous capsule, all of which will reduce the amount of bleeding in the operation and prevent tumor cells from spreading that may be caused by surgical procedures or extrusion. Furthermore, the activities and toxicities of some drugs will be decreased in liver, which can hardly be reached by intravenous injections.

Tumors are one of world's deadliest diseases nowadays. The clinical treatments such as surgery, radiotherapy, chemotherapy are effective methods to remove tumor mass. However, surgical resection can only be applied to visible tumors but have no effects on invisible subclinical focuses, tumor cells spreading to the surrounding normal tissues through the lymphatic system or bloodstream, or tumor cells infiltrating the surroundings directly. Radiotherapy is a treatment via local radiation so it is unable to kill the tumors outside the radiation area and has no effects on those insensitive tumor cells either. Chemotherapy is a systematic treatment which has poor selective inhibition effects on tumor cells and even has no effects on dormant tumor cells. For those reasons, several new methods and techniques have been developed to treat tumors in the recent years. Among them, molecular targeted therapy turns out to be a hotspot and even a trend of current researches. On the basis of tumor molecular biology, the molecular targeted therapy works by utilizing specific agents or drugs of targeted molecules to target the specific molecules associated with the cancer. This kind of treatment that targets diseased cells possesses sweeping "permanent" effects on tumors, in comparison with those three traditional therapies, surgery, radiotherapy and chemotherapy. However, the causes of tumors are various, so the treatment strategy should be designed from different aspects. The targeted therapy is the new technology applied in the current treatments of tumors and can eliminate tumors by inhibiting tumorigenesis and tumor development through various mechanisms. CN 101 336 890 A discloses slow-release microspheres containing an angiogenesis inhibitor. Sorafenib is mentioned. US 2008/020052 A1 discloses Na alginate microparticles containing paclitaxel and their use for vascular embolisation.

So far, it has not ever been reported in either China or other countries around the world that the microsphere made from sorafenib and sodium alginate can be applied to the treatment of liver cancer, renal cancer, non-small cell lung cancer, gastric cancer, ovarian cancer, prostate cancer, head and neck tumor, melanoma and other solid tumors with local vascular embolism in target region.

Thus, how to maximize sorafenib's effects on treating tumors has become an urgent technical problem of the field to be resolved.

### Disclosure of the Invention

One objective of the present invention is to provide a targeted sustained-release sodium alginate microsphere vascular embolizing agent containing sorafenib.

The above-mentioned objective can be achieved via the technical solution described below:

A targeted sustained-release sodium alginate microsphere vascular embolizing agent containing sorafenib, comprising natural carrier sodium alginate and anti-tumor drug sorafenib, where the sorafenib is encapsulated by sodium alginate and the weight ratio of the sorafenib to the sodium alginate is 1:1∼1:30.

Another objective of the present invention is to provide a method for preparing the targeted sustained-release sodium alginate microsphere vascular embolizing agent containing sorafenib.

The above-mentioned objective can be achieved via the technical solution described below:

A preparation method of targeted sustained-release sodium alginate microsphere vascular embolizing agent containing sorafenib, the steps of which are listed below:
(1) Preparation of carrier solution
   Sodium alginate is dissolved in physiological saline or water for injection proportionally to prepare a 1 wt%∼7 wt% sodium alginate carrier solution.
(2) Preparation of solidifying solution
   Calcium lactate or calcium chloride is weighted proportionally and dissolved in physiological saline or water for injection to obtain a 1 wt%∼10 wt% calcium lactate or calcium chloride solution.
(3) Preparation of drug solution
   Sorafenib is weighted proportionally and then dissolved in polyethylene glycerol 400 or dimethyl sulfoxide (DMSO) to obtain a sorafenib drug solution.
(4) Preparation of mixture of carrier solution and drug solution
   The sorafenib drug solution of Step (3) is mixed with the sodium alginate carrier solution of Step (1) by a high-speed mixer to obtain a mixture solution.
(5) Preparation of targeted sustained-release sodium alginate microsphere containing sorafenib
   The mixture solution obtained in Step (4) is reacted with the solidifying solution of Step (2) via a high-voltage electrostatic multihead microsphere generating device to obtain microspheres (or micro gel beads).

A preferred technical solution, **characterized in that** the high-voltage electrostatic multihead microsphere generating device in Step (5) comprises high-voltage generator, multiplepoint electrode, micro-infusion pump, syringe, tailor-made needle, lifting platform, and sterile glass collector.

A preferred technical solution, wherein the preparation procedure of Step (5) is described below:
1) A 10∼60 ml syringe is fitted with a tailor-made needle and then 10∼60 ml of the mixture solution obtained in Step (4) is aspirated into the syringe;
2) The syringe of Step 1) is fixed in the syringe pushing slot of the micro-infusion pump;
3) The positive interface of the high-voltage electrostatic generator is connected to the tailor-made needles of 2∼12 syringes via multiplepoint electrode; while the negative interface of the high-voltage electrostatic generator is connected, via multiplepoint electrode, to the extensions of 2∼12 b-shape stainless steel rings soaking in the solidifying solution of Step 2); the tailor-made needles are hung above the sterile glass collector which is placed on the lifting platform; the distance between the tip of the tailor-made needle and the surface of liquid in the sterile glass collector is adjusted to 5-20 cm; and once pressing start buttons on the high-voltage electrostatic generator and the micro-infusion pump, the sodium alginate mixture solution containing sorafenib is dropped into the solidifying solution in the sterile glass collector to obtain microspheres (or micro gel beads) called wet beads.

A preferred technical solution, **characterized in that** the tailor-made needle is made from stainless steel with blunt end.

A preferred technical solution, **characterized in that** the obtained microspheres (or micro gel beads) are subjected to centrifuge washing or precipitation washing and then stored in a preserving solution to obtain the sodium alginate microspheres (or micro gel beads) containing sorafenib; and the microspheres retain intact without sorafenib leaking during storage.

A preferred technical solution, **characterized in that** the preserving solution is prepared as described below:
Calcium chloride or calcium lactate is weighted proportionally and dissolved in water for injection to prepare a 3 wt%∼15 wt% preserving solution.

A preferred technical solution, **characterized in that** the particle size range of the microspheres (or micro gel beads) stored in the preserving solution is 50∼100 µm, 70∼150 µm, 100∼200 µm, 100∼300 µm, 150∼450 µm, 300-500 µm, 500∼700 µm, or 700∼900 µm.

A preferred technical solution, **characterized in that** the obtained microspheres or micro gel beads are dried via freeze drying (or oven drying) to obtained dry beads, whose particle size range is 20~60 µm, 30∼75 µm, 50∼100 µm, 70∼150 µm, 80~250 µm, 150~300 µm, 250~500 µm or 500∼700µm.

A further objective of the present invention is to provide the use of the targeted sustained-release sodium alginate microsphere vascular embolizing agent containing sorafenib.

The above-mentioned objective can be achieved via the technical solution described below:

The use of the targeted sustained-release sodium alginate microsphere vascular embolizing agent containing sorafenib for the manufacturing of medicament for the treatment of liver cancer, lung cancer, ovarian cancer, prostate cancer, head and neck tumor, melanoma and other solid tumors.

The application procedure is described below:
A catheter is inserted into the artery supplying the target organ via interventional radiolography or interventional ultrasonography and then arteriography is performed. The above-mentioned targeted sustained-release sodium alginate microsphere vascular embolizing agent containing sorafenib is chosen according to the arteriogram. When superselecting embolism, microcatheter is preferred and should be manipulated aseptically. The preserving solution in the bottle of the sodium alginate microspheres (wet beads) containing sorafenib is discarded by using syringe. The microspheres are washed with the same amount of physiological saline for 3 times, or are frstly transferred to a sterile bowl from the bottle and then washed with 50∼100 ml physiological saline for 1∼3 times. After discarding the washing fluid, an appropriate amount of contrast agent or the diluted contrast agent is added and mixed with the microspheres to make the microspheres fully suspend in the contrast medium, which is then injected into the focus slowly, in accordance with specific conditions, through the catheter under fluoroscopic control. When the flow of the contrast medium slows down apparently, the embolization is completed. Arteriography is performed once again to evaluate the effectiveness of embolization.

If dry beads are applied, the mircrospheres cannot be used until turning back into wet beads by being soaked in physiological saline half an hour in advance of application.

### Beneficial effects

By altering the dosage form as well as changing the route of administration, the targeted sustained-release sodium alginate microsphere vascular embolizing agent containing sorafenib of the present invention enables the targeted drugs to be directed to the target region and then to have a rapid, long and focused effect on the cancer tissue. So its advantages lie in good targets, excellent therapeutic effects, negligible harm to normal tissues while killing cancer cells, low toxicity, small amount of required drugs and low treating cost.

The targeted sustained-release sodium alginate microsphere vascular embolizing agent containing sorafenib enhances therapeutic effects by utilizing new techniques to facilitate sorafenib to reach the target region rapidly, to be released sustainably and focused around cancer cells, which reduces the cycle of the drugs, required doses, damage of normal cells and toxicity.

Currently, there are some problems associated with oral administration of sorafenib, including low bioavailability, large doses required, and high toxicity, all of which cause that the medical cost is too high to be acceptable to either doctors or patients. The combination of anti-cancer drug and embolizing agent results in a combined effect when positioning the target region; while the separate normal administrations of these two drugs at the same time have no such an effect. The microsphere encapsulating the targeted drug sorafenib and the arterial vascular embolizing agent allows the concentration of drugs to be maintained in the local tissues for a longer time. The targeted sustained-release sodium alginate microsphere vascular embolizing agent containing sorafenib results in a focused effect by avoiding the first-pass effect whereby the drug is damaged and excreted via systemic circulation and in liver, kidney and other organs, reducing the probability of failure that drug binds to plasma proteins, prolonging the functioning time of the drug, all of which may conquer the defects of oral administration, intravenous chemotherapy and simple drug infusion, including short retention time in tumor tissues, fast clearance from tumors and inadequate exposure of drug to tumor cells. The clinical pharmacokinetics studies suggests that the quantity of killed cancer cells is increased by 10 to 100 times and the therapeutic effect is doubled when the concentration of anti-cancer drug is doubled within a certain range in the local tissue. Since the targeted sustained-release sodium alginate microsphere vascular embolizing agent containing sorafenib has been developed successfully, the traditional routes of drug administration will be changed and patients would therefore enjoy efficiency, comfort and convenience which are brought by the new-type agent. It will also play an indispensable role in treating solid tumors.

The inventors of the present invention found that the 2∼12 micro-infusion devices in the high-voltage electrostatic multihead microsphere generating device allows more uniform microspheres to be prepared, the yield to be increased, and the microspheres of difference particle size to be produced at the same time.

Hereinafter, the present invention will be further described in the following embodiments. However, these embodiments are not intended to restrict the scope of the present invention.

### Detailed Description of the Preferred Embodiments

### Example 1

### 1. Preparation before encapsulating

Treatment of glass wares: The clean glass wares were dried out in the air and then baked in high-temperature oven at 260 °C for 3 hours to kill bacteria and remove pyrogens.

### 2. Preparation of reagents

(1) Preparation of anti-tumor drug sorafenib solution
   10 mg of commercial sorafenib was weighted and added into the above-mentioned glass ware. An appropriate amount of polyethylene glycerol 400 was then dropped till the sorafenib was fully dissolved to obtain 20 ml of sorafenib solution.
(2) Preparation of sodium alginate solution
   3L of 2 wt% sodium alginate solution was prepared by adding physiological saline into sodium alginate while stirring till sodium alginate was fully dissolved.
(3) Preparation of solidifying solution
   Adequate calcium chloride was weighted and dissolved in physiological saline to prepare a 3 wt% calcium chloride solution.
(4) Preparation of preserving solution
   Adequate calcium chloride was weighted and dissolved in water for injection to prepare a 3 wt% calcium chloride solution, i.e. the preserving solution.
(5) 20 ml of the above-mentioned sorafenib drug solution was mixed with 3 L of the alginate solution by a high-speed mixer to obtain the mixture solution containing sodium alginate and sorafenib.

### 3. Preparation of sodium alginate microsphere containing sorafenib

(1) Two 10 ml syringes were fitted with tailor-made needles, respectively, and then the mixture of sorafenib solution and sodium alginate solution was aspirated into the syringes in at least 151 times, respectively.
(2) The syringes mentioned in Step (1) of the preparing process of microsphere were fixed in the syringe pushing slot of the micro-infusion pump.
(3) The positive interface of the high-voltage electrostatic generator was connected to the tailor-made needles of the 2 syringes via multiplepoint electrode; while the negative interface of the high-voltage electrostatic generator was connected, via multiplepoint electrode, to the extensions of the two b-shape stainless steel rings soaking in the solidifying solution of Step (2); the tailor-made needles were hung above the sterile glass collector which was placed on the lifting platform; the distance between the tip of the tailor-made needles and the surface of liquid in the sterile glass collector was adjusted to 12 cm; and once pressing the start buttons of the high-voltage electrostatic generator and the micro-infusion pump, the sodium alginate mixture solution containing sorafenib was dropped into the solidifying solution in the sterile glass collector to obtain microspheres (or micro gel beads) called wet beads. The tailor-made needle was made from stainless steel with blunt end.
(4) Washing: the obtained microspheres (or micro gel beads) were subjected to centrifuge washing or precipitation washing and then stored in the 3 wt% preserving solution. During storage, the microspheres retain intact without sorafenib leaking.
(5) The particle size of the microspheres (or micro gel beads) stored in the preserving solution ranged from 70 to 150 µm.
(6) The obtained sodium alginate microspheres or micro gel beads containing sorafenib were dried out via freeze drying to obtain dry beads, whose particle size ranged from 30 to 75 µm.

The microspheres can be used right after turning back into wet beads by being soaked in physiological saline half an hour in advance of application.

### 4. Application to treating patients via targeted vessel embolisation

For the patient suffering from liver cancer, a catheter was inserted into the artery supplying the target organ via interventional radiolography or interventional ultrasonography and then arteriography was performed. The above-mentioned targeted sustained-release sodium alginate microsphere vascular embolizing agent containing sorafenib is chosen according to the arteriogram. When superselecting embolism, microcatheter would be preferred and should be manipulated aseptically. The calcium chloride solution in the bottle of the sodium alginate microspheres (wet beads) containing sorafenib was discarded by using syringe. The microspheres were washed with the same amount of physiological saline for 3 times, or were firstly transferred to a sterile bowl from the bottle and then washed with 50∼100 ml physiological saline for 1∼3 times. After discarding the washing fluid, an appropriate amount of contrast agent or diluted contrast agent was added and mixed with the microspheres to make the microspheres fully suspend in the contrast medium, which was then injected into the focus slowly, in accordance with specific conditions, through the catheter under fluoroscopic control. When the flow of the contrast medium slowed down apparently, the embolization was completed. Arteriography was performed once again to evaluate the effectiveness of embolization.

### Example 2

### 1. Preparation before encapsulating

Treatment of glass wares: The clean glass wares were dried out in the air and then baked in high-temperature oven at 260 °C for 3 hours to kill bacteria and remove pyrogens.

### 2. Preparation of reagents

(1) Preparation of anti-tumor drug sorafenib solution
   0.62 g of commercial sorafenib was weighted and added into the above-mentioned glass ware. An appropriate amount of dimethyl sulfoxide (DMSO) was then dropped till the sorafenib was fully dissolved to obtain 500 ml of sorafenib solution.
(2) Preparation of sodium alginate solution
   45 L of 1 wt% sodium alginate solution was prepared by adding physiological saline into sodium alginate while stirring till sodium alginate was fully dissolved.
(3) Preparation of solidifying solution
   Adequate calcium lactate was weighted and dissolved in physiological saline to prepare a 1 wt% calcium lactate solution.
(4) Preparation of preserving solution
   Adequate calcium chloride was weighted and dissolved in water for injection to prepare a 8 wt% calcium chloride solution, i.e. the preserving solution.
(5) 500 ml of the above-mentioned sorafenib solution was mixed with 45 L of the sodium alginate solution by a high-speed mixer to obtain the mixture solution containing sodium alginate and sorafenib.

### 3. Preparation of sodium alginate microsphere containing sorafenib

(1) Twelve 60 ml syringes were fitted with tailor-made needles and then the mixture of sorafenib solution and sodium alginate solution was aspirated into the syringes in at least 63 times.
(2) The syringes mentioned in Step (1) of the preparing process of microsphere were fixed in the syringe pushing slot of the microinfusion pump. The parameters of the pump were also adjusted.
(3) The positive interface of the high-voltage electrostatic generator was connected to the tailor-made needles of the 12 syringes via multiplepoint electrode; while the negative interface of the high-voltage electrostatic generator was connected, via multiplepoint electrode, to the extensions of the 12 b-shape stainless steel rings soaking in the solidifying solution of Step (2); the tailor-made needles were hung above the sterile glass collector which was placed on the lifting platform; the distance between the tip of the tailor-made needles and the surface of liquid in the sterile glass collector was adjusted to 5 cm; and once pressing the start buttons of the high-voltage electrostatic generator and the micro-infusion pump, the sodium alginate mixture solution containing sorafenib was dropped into the solidifying solution in the sterile glass collector to obtain microspheres (or micro gel beads) called wet beads. The tailor-made needle was made from stainless steel with blunt end.
(4) Washing: the obtained microspheres (or micro gel beads) were subjected to centrifuge washing or precipitation washing and then stored in the 8 wt% preserving solution. During storage, the microspheres retain intact without sorafenib leaking.
(5) The particle size of the microspheres (or micro gel beads) stored in the preserving solution ranged from 300 to 500 µm.
(6) The obtained sodium alginate microspheres (or micro gel beads) containing sorafenib were dried out via freeze drying (or oven drying) to obtain dry beads, whose particle size ranged from 150 to 300 µm.

The mircrospheres can be used right after turning back into wet beads by being soaked in physiological saline half an hour in advance of application.

### 4. Application to treating patients via targeted vessel embolisation

For the patient suffering from renal cancer, a catheter was inserted into the artery supplying the target organ via interventional radiolography or interventional ultrasonography and then arteriography was performed. The above-mentioned targeted sustained-release sodium alginate microsphere vascular embolizing agent containing sorafenib is chosen according to the arteriogram. When superselecting embolism, microcatheter would be preferred and should be manipulated aseptically. The calcium chloride solution in the bottle of the sodium alginate microspheres (wet beads) containing sorafenib was discarded by using syringe. The microspheres were washed with the same amount of physiological saline for 3 times, or were firstly transferred to a sterile bowl from the bottle and then washed with 50∼100 ml physiological saline for 1∼3 times. After discarding the washing fluid, an appropriate amount of contrast agent or diluted contrast agent was added and mixed with the microspheres to make the microspheres fully suspend in the contrast medium, which was then injected into the focus slowly, in accordance with specific conditions, through the catheter under fluoroscopic control. When the flow of the contrast medium slowed down apparently, the embolization was completed. Arteriography was performed once again to evaluate the effectiveness of embolization.

### Example 3

### 1. Preparation before encapsulating

Treatment of glass wares: The clean glass wares were dried out in the air and then baked in high-temperature oven at 260 °C for 3 hours to kill bacteria and remove pyrogens.

### 2. Preparation of reagents

(1) Preparation of anti-tumor drug sorafenib solution
   6.9 mg of commercial sorafenib was weighted and added into the above-mentioned glass ware. An appropriate amount of dimethyl sulfoxide (DMSO) was then dropped till the sorafenib was fully dissolved to obtain 30 ml of sorafenib solution.
(2) Preparation of sodium alginate solution
   2,000 ml of 7 wt% sodium alginate solution was prepared by adding physiological saline into sodium alginate while stirring till sodium alginate was fully dissolved.
(3) Preparation of solidifying solution
   Adequate calcium lactate was weighted and dissolved in water for injection to prepare a 10 wt% calcium lactate solution.
(4) Preparation of preserving solution
   Adequate calcium lactate was weighted and dissolved in water for injection to prepare a 15 wt% preserving solution.
(5) 30 ml of the above-mentioned sorafenib solution was mixed with 2,000 ml of the sodium alginate solution by a high-speed mixer to obtain the mixture solution containing sodium alginate and sorafenib.

### 3. Preparation of sodium alginate microsphere containing sorafenib

(1) Ten 50 ml syringes were fitted with tailor-made needles and then the mixture of sorafenib solution and sodium alginate solution was aspirated into the syringe in at least 4 times.
(2) The syringes mentioned in Step (1) of the preparing process of microsphere were fixed in the syringe pushing slot of the microinfusion pump.
(3) The positive interface of the high-voltage electrostatic generator was connected to the tailor-made needles of the 10 syringes via multiplepoint electrode; while the negative interface of the high-voltage electrostatic generator was connected, via multiplepoint electrode, to the extensions of the 10 b-shape stainless steel rings soaking in the solidifying solution of Step (2); the tailor-made needles were hung above the sterile glass collector which was placed on the lifting platform; the distance between the tip of the tailor-made needles and the surface of liquid in the sterile glass collector was adjusted to 5 cm; and once pressing the start buttons of high-voltage electrostatic generator and the micro-infusion pump, the sodium alginate mixture solution containing sorafenib was dropped into the solidifying solution in the sterile glass collector to obtain microspheres (or micro gel beads) called wet beads. The tailor-made needle was made from stainless steel with blunt end.
(4) Washing: the obtained microspheres (or micro gel beads) were subjected to centrifuge washing or precipitation washing and then stored in the 15 wt% preserving solution. During storage, the microspheres retain intact without sorafenib leaking.
(5) The particle size of the microspheres (or micro gel beads) stored in the preserving solution ranged from 500 to 700 µm.
(6) The obtained sodium alginate microspheres (or micro gel beads) containing sorafenib were dried out via oven drying to obtain dry beads, whose particle size ranged from 250 to 500 µm.

The mircrospheres can be used right after turning back into wet beads by being soaked in physiological saline half an hour in advance of application.

### 4. Application to treating patients via targeted vessel embolisation

For the patient suffering from lung cancer, a catheter was inserted into the artery supplying the target organ via interventional radiolography or interventional ultrasonography and then arteriography was performed. The above-mentioned targeted sustained-release sodium alginate microsphere vascular embolizing agent containing sorafenib is chosen according to the arteriogram. When superselecting embolism, microcatheter would be preferred and should be manipulated aseptically. The calcium chloride solution in the bottle of the sodium alginate microspheres (wet beads) containing sorafenib was discarded by using syringe. The microspheres were washed with the same amount of physiological saline for 3 times, or were firstly transferred to a sterile bowl from the bottle and then washed with 50∼100 ml physiological saline for 1∼3 times. After discarding the washing fluid, an appropriate amount of contrast agent or diluted contrast agent were added and mixed with the microspheres to make the microspheres fully suspend in the contrast medium, which was then injected into the focus slowly, in accordance with specific conditions, through the catheter under fluoroscopic control. When the flow of the contrast medium slowed down apparently, the embolization was completed. Arteriography was performed once again to evaluate the effectiveness of embolization.

## Claims

1. A targeted sustained-release sodium alginate microsphere vascular embolizing agent containing sorafenib comprising natural carrier sodium alginate and anti-tumor drug sorafenib, wherein the sorafenib is encapsulated by the sodium alginate and the weight ratio of the sorafenib to the sodium alginate is 1:1∼1:30.

2. A preparation method of the targeted sustained-release sodium alginate microsphere vascular embolizing agent containing sorafenib according to Claim 1, wherein the preparation method comprises steps as below:
(1) Preparation of carrier solution
Sodium alginate is dissolved in physiological saline or water for injection proportionally to prepare a 1 wt%∼7 wt% solution then a sodium alginate carrier solution is obtained;
(2) Preparation of solidifying solution
Calcium lactate or calcium chloride is weighted and dissolved in physiological saline or water for injection proportionally to prepare a 1 wt%∼10 wt% calcium lactate or calcium chloride solution;
(3) Preparation of drug solution
Sorafenib is weighted proportionally and then dissolved in polyethylene glycerol 400 or dimethyl sulfoxide to obtain a sorafenib drug solution;
(4) Preparation of the mixture of carrier solution and drug solution
The sorafenib drug solution obtained in Step (3) is mixed with the sodium alginate carrier solution obtained in Step (1) by a high-speed mixer to obtain a mixture solution;
(5) Preparation of targeted sustained-release sodium alginate microsphere containing sorafenib
The mixture solution obtained in Step (4) is reacted with the solidifying solution obtained in Step (2) via a high-voltage electrostatic multihead microsphere generating device to obtain microspheres (or micro gel beads).

3. The preparation method of the targeted sustained-release sodium alginate microsphere vascular embolizing agent containing sorafenib according to Claim 2, **characterized in that** the high-voltage electrostatic multihead microsphere generating device in Step (5) comprises high-voltage generator, multiplepoint electrode, micro-infusion pump, syringe, tailor-made needle, lifting platform, and sterile glass collector.

4. The preparation method of the targeted sustained-release sodium alginate microsphere vascular embolizing agent containing sorafenib according to Claim 3, **characterized in that** the preparation procedure of Step (5) is described below:
1) A 10∼60 ml syringe is fitted with a tailor-made needle and then 10∼60 ml of the mixture solution obtained in Step (4) is aspirated into the syringe;
2) The syringe in Step 1) is fixed in the syringe pushing slot of the micro-infusion pump;
3) The positive interface of the high-voltage electrostatic generator is connected to the tailor-made needles of 2∼12 syringes via multiplepoint electrode; while the negative interface of high-voltage generator is connected, via multiplepoint electrode, to the extensions of 2∼12 b-shape stainless steel rings soaking in the solidifying solution obtained in Step (2); the tailor-made needles are hung above the sterile glass collector which is placed on the lifting platform; the distance between the tip of the tailor-made needle and the surface of liquid in the sterile glass collector is adjusted to 5-20 cm; and once pressing the start buttons of the high-voltage electrostatic generator and the micro-infusion pump, the sodium alginate mixture solution containing sorafenib is dropped into the solidifying solution in the sterile glass collector to obtain the microspheres (or micro gel beads) called wet beads.

5. The preparation method of the targeted sustained-release sodium alginate microsphere vascular embolizing agent containing sorafenib according to Claim 4, **characterized in that** the tailor-made needle is made from stainless steel with blunt end.

6. The preparation method of the targeted sustained-release sodium alginate microsphere vascular embolizing agent containing sorafenib according to Claim 5, **characterized in that** the obtained microspheres or micro gel beads are subjected to centrifuge washing or precipitation washing and then stored in a preserving solution to obtain the sodium alginate microspheres or micro gel beads containing sorafenib; and the microspheres retain intact without sorafenib leaking during storage.

7. The preparation method of the targeted sustained-release sodium alginate microsphere vascular embolizing agent containing sorafenib according to Claim 6, **characterized in that** the preserving solution is prepared as below:
calcium chloride or calcium lactate is weighted proportionally and dissolved in water for injection to prepare a 3 wt%∼15 wt% preserving solution.

8. The preparation method of the targeted sustained-release sodium alginate microsphere vascular embolizing agent containing sorafenib according to Claim 7, **characterized in that** the particle size range of the microspheres or micro gel beads stored in the preserving solution is 50∼100 µm, 70∼150 µm, 100∼200 µm, 100∼300µm, 150∼450µm, 300∼500µm, 500∼700µm, or 700~900 µm.

9. The preparation method of the targeted sustained-release sodium alginate microsphere vascular embolizing agent containing sorafenib according to Claim 8, **characterized in that** the obtained microspheres or micro gel beads are dried via freeze drying or oven drying to obtain dry beads, whose particle size range is 20~60 µm, 30∼75 µm, 50∼100 µm, 70∼150 µm, 80∼250 µm, 150∼300 µm, 250∼500 µm or 500∼700µm.

10. The use of the targeted sustained-release sodium alginate microsphere vascular embolizing agent containing sorafenib according to Claim 1 for the manufacturing of medicament for the treatment of solid tumors including liver cancer, lung cancer, renal cancer, ovarian cancer, prostate cancer, and head and neck tumor.

## Patentansprüche

1. Gezielte Retard-Natriumalginat-Mikrokügelchen mit einem Gefäßokklusionsmittel enthaltend Sorafenib umfassend als natürlichen Träger Natriumalginat und den Antitumorwirkstoff Sorafenib, wobei das Sorafenib durch das Natriumalginat eingekapselt ist und das Gewichtsverhältnis von Sorafenib zu Natriumalginat 1:1 ∼ 1:30 ist.

2. Verfahren zur Herstellung des gezielten Retard-Natriumalginat-Mikrokügelchens mit einem Gefäßokklusionsmittel enthaltend Sorafenib gemäß Anspruch 1, wobei das Herstellverfahren unten stehende Schritte umfasst:
(1) Herstellung der Trägerlösung
Natriumalginat wird in physiologischer Salzlösung oder Wasser für Injektionszwecke anteilsmäßig gelöst, um eine 1 Gew.% ∼ 7 Gew.% Lösung herzustellen, wodurch eine Natriumalginat-Trägerlösung erhalten wird;
(2) Herstellung der verfestigenden Lösung
Calciumlactat oder Calciumchlorid wird ausgewogen und in physiologischer Salzlösung oder Wasser für Injektionszwecke proportional gelöst, um eine 1 Gew.% ∼ 10 Gew.% Calciumlactat- oder Calciumchlorid-Lösung herzustellen;
(3) Herstellen der Arzneimittellösung
Sorafenib wird proportional abgewogen und dann in Polyethylenglycerol 400 oder Dimethylsulfoxid gelöst, um eine Sorafenib-Arzneimittellösung zu erhalten;
(4) Herstellen der Mischung aus Trägerlösung und Arzneimittellösung Die Sorafenib-Arzneimittellösung erhalten in Schritt (3) wird mit der Natriumalginat-Trägerlösung erhalten in Schritt (1) durch einen Hochgeschwindigkeitsmischer gemischt, um eine Mischungslösung zu erhalten;
(5) Herstellung eines gezielten Retard-Natriumalginat-Mikrokügelchens enthaltend Sorafenib
Die Mischung erhalten in Schritt (4) wird mit der verfestigenden Lösung erhalten in Schritt (2) in einer elektrostatischen Mikrokügelchen-bildenden Hochspannungsmehrkopfvorrichtung umgesetzt, um Mikrokügelchen (oder Mikrogelkügelchen) zu erhalten.

3. Verfahren zur Herstellung des gezielten Retard-Natriumalginat-Mikrokügelchens mit einem Gefäßokklusionsmittel enthaltend Sorafenib gemäß Anspruch 2, wobei die elektrostatische Mikrokügelchen-bildende Hochspannungsmehrkopfvorrichtung in Schritt (5) einen Hochspannungsgenerator, Mehrpunktelektrode, Mikroinfusionspumpe, Spritze, maßgeschneiderte Nadel, Hebebühne und sterilen Glassammler umfasst.

4. Verfahren zur Herstellung des gezielten Retard-Natriumalginat-Mikrokügelchens mit einem Gefäßokklusionsmittel enthaltend Sorafenib gemäß Anspruch 3, wobei das Herstellungsverfahren von Schritt (5) unten beschrieben wird:
1) Eine 10~60 mL Spritze wird mit einer maßgeschneiderten Nadel ausgestattet und dann 10~60 mL einer Mischung erhalten in Schritt (4) in die Spritze gesaugt;
2) Die Spritze in Schritt 1 wird in die Spritzenauslösungsvorrichtung der Mikroinfusionspumpe fixiert;
3) Die positive Schnittstelle des elektrostatischen Hochspannungsgenerators ist mit der maßgeschneiderten Nadel der 2~12 Spritzen über die Mehrpunktelektrode verbunden; während die negative Schnittstelle des Hochspannungsgenerators über die Mehrpunktelektrode an die Verlängerungen der 2∼12 b-förmigen Edelstahlringe verbunden ist, die in der verfestigenden Lösung erhalten nach Schritt (2) saugen; die maßgeschneiderten Nadeln werden über dem sterilen Glassammler aufgehängt, der über der Hebebühne angeordnet ist; die Strecke zwischen der Spitze der maßgeschneiderten Nadel und der Flüssigkeitsoberfläche in dem sterilen Glassammler wird auf 5-20 cm eingestellt; und sobald die Startknöpfe des elektrostatischen Hochspannungsgenerators und der Mikroinfusionspumpe gedrückt werden, tropft die Natriumalginat-Mischungslösung enthaltend Sorafenib in die verfestigende Lösung in den sterilen Glassammler, um Mikrokügelchen (oder Mikrogelkügelchen) genannt nasse Beads zu erhalten.

5. Verfahren zur Herstellung des gezielten Retard-Natriumalginat-Mikrokügelchens mit einem Gefäßokklusionsmittel enthaltend Sorafenib gemäß Anspruch 4, wobei die maßgeschneiderte Nadel aus Edelstahl mit einem stumpfen Ende hergestellt ist.

6. Verfahren zur Herstellung des gezielten Retard-Natriumalginat-Mikrokügelchens mit einem Gefäßokklusionsmittel enthaltend Sorafenib gemäß Anspruch 5, wobei die erhaltenen Mikrokügelchen oder Mikrogelkügelchen in einer Zentrifuge gewaschen werden oder durch Präzipitation gewaschen werden und dann in einer konservierenden Lösung gelagert werden, um Natriumalginat-Mikrokügelchen oder Mikrogelkügelchen enthaltend Sorafenib zu erhalten; und die Mikrokügelchen intakt bleiben, ohne dass Sorafenib während der Lagerung austritt.

7. Verfahren zur Herstellung des gezielten Retard-Natriumalginat-Mikrokügelchens mit einem Gefäßokklusionsmittel enthaltend Sorafenib gemäß Anspruch 6, wobei die konservierende Lösung wie unten hergestellt wird:
Calciumchlorid oder Calciumlactat wird proportional abgewogen und in Wasser für Injektionszwecke gelöst, um eine 3 Gew.%∼15 Gew.% konservierende Lösung herzustellen.

8. Verfahren zur Herstellung des gezielten Retard-Natriumalginat-Mikrokügelchens mit einem Gefäßokklusionsmittel enthaltend Sorafenib gemäß Anspruch 7, wobei der Bereich der Partikelgröße der Mikrokügelchen oder Mikrogelkügelchen, die in der konservierenden Lösung gelagert werden 50∼100 µm, 70∼150 µm, 100∼200 µm, 100∼300 µm, 150∼450 µm, 300-500 µm, 500-700 µm oder 700-900 µm ist.

9. Verfahren zur Herstellung des gezielten Retard-Natriumalginat-Mikrokügelchens mit einem Gefäßokklusionsmittel enthaltend Sorafenib gemäß Anspruch 8, wobei die erhaltenen Mikrokügelchen oder Mikrogelkügelchen durch Gefriertrocknen oder Ofentrocknen getrocknet werden, um trockene Kügelchen zu erhalten, deren Bereich der Partikelgröße 20-60 µm, 30-75 µm, 50∼100 µm, 70∼150 µm, 80∼250 µm, 150∼300 µm, 250-500 µm oder 500-700 µm ist.

10. Die Verwendung des gezielten Retard-Natriumalginat-Mikrokügelchens mit einem Gefäßokklusionsmittel enthaltend Sorafenib gemäß Anspruch 1 zur Herstellung eines Medikaments für die Behandlung von soliden Tumoren beinhaltend Leberkrebs, Lungenkrebs, Nierenkrebs, Eierstockkrebs, Prostatakrebs, und Kopf- und Halskrebs.

## Revendications

1. Agent d'embolisation vasculaire, à microsphères d'alginate de sodium, à libération prolongée et ciblée, contenant du sorafénib, comprenant le véhicule naturel alginate de sodium et le médicament anti-tumoral sorafénib, dans lequel le sorafénib est encapsulé par l'alginate de sodium et le rapport pondéral du sorafénib à l'alginate de sodium est de 1:1∼1:30.

2. Procédé de préparation de l'agent d'embolisation vasculaire, à microsphères d'alginate de sodium, à libération prolongée et ciblée, contenant du sorafénib, selon la revendication **1,** dans lequel le procédé de préparation comprend les étapes ci-dessous :
(1) Préparation d'une solution véhicule
De l'alginate de sodium est dissous dans du sérum physiologique ou dans de l'eau pour injection proportionnellement de façon à préparer une solution de 1 % en poids - 7 % en poids, puis une solution véhicule d'alginate de sodium est obtenue ;
(2) Préparation d'une solution de solidification
Du lactate de calcium ou du chlorure de calcium est pesé et dissous dans du sérum physiologique ou dans de l'eau pour injection proportionnellement de façon à préparer une solution de lactate de calcium ou de chlorure de calcium de 1 % en poids - 10 % en poids ;
(3) Préparation d'une solution de médicament
Du sorafénib est pesé proportionnellement et ensuite dissous dans du polyéthylène glycérol 400 ou dans du diméthylsulfoxyde pour obtenir une solution de médicament sorafénib ;
(4) Préparation du mélange de la solution véhicule et de la solution de médicament La solution de médicament sorafénib obtenue à l'étape (3) est mélangée avec la solution véhicule d'alginate de sodium obtenue à l'étape (1) par un mélangeur à grande vitesse pour obtenir une solution de mélange ;
(5) Préparation d'une microsphère d'alginate de sodium, à libération prolongée et ciblée contenant du sorafénib
La solution de mélange obtenue à l'étape (4) est mise en réaction avec la solution de solidification obtenue à l'étape (2) par l'intermédiaire d'un dispositif générateur de microsphère à plusieurs têtes, électrostatique, à haute tension, pour obtenir des microsphères (ou des microbilles de gel).

3. Procédé de préparation de l'agent d'embolisation vasculaire, à microsphères d'alginate de sodium, à libération prolongée et ciblée, contenant du sorafénib, selon la revendication **2, caractérisé en ce que** le dispositif générateur de microsphère à plusieurs têtes, électrostatique, à haute tension de l'étape (5) comprend un générateur haute tension, une électrode multi-points, une pompe à micro-infusion, une seringue, une aiguille faite sur mesure, une plateforme d'élévation et un collecteur en verre stérile.

4. Procédé de préparation de l'agent d'embolisation vasculaire, à microsphères d'alginate de sodium, à libération prolongée et ciblée, contenant du sorafénib, selon la revendication **3, caractérisé en ce que** le mode opératoire de préparation de l'étape (5) est décrit ci-dessous :
1) Une seringue de 10 ∼ 60 ml est montée dans une aiguille faite sur mesure et ensuite 10 ∼ 60 ml de la solution de mélange obtenue dans l'étape (4) sont aspirés dans la seringue ;
2) La seringue de l'étape 1) est fixée dans la fente du pousse-seringue de la pompe à micro-infusion ;
3) L'interface positive du générateur électrostatique haute tension est connectée aux aiguilles faites sur mesure de 2 ∼ 12 seringues par l'intermédiaire d'une électrode multi-points ; alors que l'interface négative du générateur haute tension est connectée, par l'intermédiaire d'une électrode multi-points, aux extensions de 2 ∼ 12 anneaux en acier inoxydable en forme de b trempant dans la solution de solidification obtenue à l'étape (2) ; les aiguilles faites sur mesure sont suspendues au-dessus du collecteur en verre stérile qui est placé sur la plateforme d'élévation ; la distance entre l'extrémité de l'aiguille faite sur mesure et la surface du liquide dans le collecteur en verre stérile est ajustée à une valeur de 5 - 20 cm ; et lorsque les boutons de démarrage du générateur électrostatique haute tension et de la pompe à micro-infusion sont pressés, la solution de mélange d'alginate de sodium contenant du sorafénib est déposée dans la solution de solidification dans le collecteur en verre stérile pour obtenir les microsphères (ou les microbilles de gel) appelées billes mouillées.

5. Procédé de préparation de l'agent d'embolisation vasculaire, à microsphères d'alginate de sodium, à libération prolongée et ciblée, contenant du sorafénib, selon la revendication **4, caractérisé en ce que** l'aiguille faite sur mesure est faite d'acier inoxydable avec un bout émoussé.

6. Procédé de préparation de l'agent d'embolisation vasculaire, à microsphères d'alginate de sodium, à libération prolongée et ciblée, contenant du sorafénib, selon la revendication **5, caractérisé en ce que** les microsphères ou les microbilles de gel obtenues sont soumises à un lavage par centrifugation ou à un lavage par précipitation et ensuite stockées dans une solution de conservation pour obtenir les microsphères ou les microbilles de gel d'alginate de sodium contenant du sorafénib ; et les microsphères restent intactes sans que le sorafénib ne fuie pendant le stockage.

7. Procédé de préparation de l'agent d'embolisation vasculaire, à microsphères d'alginate de sodium, à libération prolongée et ciblée, contenant du sorafénib, selon la revendication **6, caractérisé en ce que** la solution de conservation est préparée comme ci-dessous :
du chlorure de calcium ou du lactate de calcium est pesé proportionnellement et dissous dans de l'eau pour injection pour préparer une solution de conservation de 3 % en poids - 15 % en poids.

8. Procédé de préparation de l'agent d'embolisation vasculaire, à microsphères d'alginate de sodium, à libération prolongée et ciblée, contenant du sorafénib, selon la revendication 7, **caractérisé en ce que** la gamme de taille de particule des microsphères ou des microbilles de gel stockées dans la solution de conservation est de 50 ∼ 100 µm, de 70∼150 µm, de 100 - 200 µm, de 100 - 300 µm, de 150 - 450 µm, de 300 ∼ 500 µm, de 500 ∼ 700 µm ou de 700 ∼ 900 µm.

9. Procédé de préparation de l'agent d'embolisation vasculaire, à microsphères d'alginate de sodium, à libération prolongée et ciblée, contenant du sorafénib, selon la revendication **8, caractérisé en ce que** les microsphères ou les microbilles de gel obtenues sont séchées par lyophilisation ou par séchage au four pour obtenir des billes sèches, dont la gamme de taille de particule est de 20 ~ 60 µm, de 30 ~ 75 µm, de 50 ∼ 100 µm, de 70 ∼ 150 µm, de 80 ~ 250 µm, de 150 ~ 300 µm, de 250 ∼ 500 µm ou de 500 ∼ 700 µm.

10. Utilisation de l'agent d'embolisation vasculaire, à microsphères d'alginate de sodium, à libération prolongée et ciblée, contenant du sorafénib, selon la revendication 1, pour la fabrication d'un médicament pour le traitement de tumeurs solides, incluant le cancer du foie, le cancer du poumon, le cancer des reins, le cancer des ovaires, le cancer de la prostate et une tumeur de la tête et du cou.
